Europäisches Patentamt

European Patent Office

Office européen des brevets

(11) Publication number: **0 101 715**
**B1**

# EUROPEAN PATENT SPECIFICATION

(45) Date of publication of patent specification: **10.12.86**

(51) Int. Cl.⁴: **C 12 N 5/00, C 12 M 3/00**

(21) Application number: **83900862.0**

(22) Date of filing: **21.01.83**

(86) International application number:
**PCT/US83/00090**

(87) International publication number:
**WO 83/02953 01.09.83 Gazette 83/20**

(54) PROCESS AND APPARATUS FOR CONTROLLING PATTERNS OF CHEMICALS STIMULI ADMINISTRATION TO BIOLOGICAL TISSUE.

(30) Priority: **19.02.82 US 350135**

(43) Date of publication of application:
**07.03.84 Bulletin 84/10**

(45) Publication of the grant of the patent:
**10.12.86 Bulletin 86/50**

(84) Designated Contracting States:
**DE FR GB SE**

(56) References cited:
**FR-A-2 293 488**
**US-A-3 926 736**
**US-A-4 064 015**
**US-A-4 167 450**

**P.E. BOTHAMLEY et al.: "Computer control of an isolated heart preparation for biomedical research", pages 299-305. DIGITAL COMPUTER APPLICATIONS TO PROCESS CONTROL, Proceedings of the 5th IFAC/IFIP International Conference, The Hague, the Netherlands, June 14-17, 1977, North-Holland Publishing Company**

(73) Proprietor: **ENDOTRONICS INC.**
**P.O.Box 32315**
**Minneapolis MN 55432-0315 (US)**

(72) Inventor: **GRUENBERG, Micheal L.**
**6726 North Sheridan Road Suite 308**
**CHicago, IL 60626 (US)**

(74) Representative: **Vossius & Partner**
**Siebertstrasse 4 P.O. Box 86 07 67**
**D-8000 München 86 (DE)**

# Description

## 1. Field of the invention

The present invention relates to processes that supply chemical stimuli to a biological tissue isolated from its natural nutrient supply. In particular, it relates to a process that supplies the chemical stimuli such that the concentration of the chemical stimuli affecting the tissue is known at each and every point in time and where the parameters of various amplitudes, durations, rate dynamics, periodicities, frequency of oscillations, notch, plateau, bimodal, and phase relationships of chemical stimuli can be controlled and manipulated.

## 2. Description of the prior art

The maintenance of biological tissue, such as organs, isolated from their natural nutrient supply is of great importance. A significant amount of research has been done with regard to organs that have been removed from a body. The research is quite varied and ranges from simply trying to keep the particular organ alive outside of the body to studying the complex responses of the isolated organ to various chemical stimuli.

Typically, the organ is placed in a chamber and supported by some type of culture medium. Initially, the system used to keep an organ alive was a static one wherein the nutrients were initially mixed in the culture medium and were never replenished. This type of system was limited in its ability to sustain an organ in a biologically alive state since the culture medium very quickly became contaminated from the organ's waste products and natural secretions.

Deficiencies of the static system led to the development of a dynamic system wherein the culture medium and the chamber holding the organ was replenished and was capable of sustaining biological life for a significantly extended period of time. These systems are commonly referred to as perfusion, perifusion or superfusion systems and have significantly increased the time available for studying organs and other tissue outside of the body.

The perifusion system is a system wherein the organ is surrounded by a culture medium containing nutrients that are absorbed by the organ. The perfusion system supplies nutrients to the organ through the organ's cardiovascular system. The superfusion system includes a system that both perfuses and perifuses nutrients to the organ.

Typically, the dynamic systems include a chamber wherein the organ or other tissue is placed. The chamber has an inlet for providing nutrient-containing culture medium and an outlet removing culture medium at a rate which preferably keeps the volume of the culture medium constant within the chamber. The dynamic systems have several advantages. First, conditions in the dynamic systems more closely resemble conditions within the body than the conditions in a static system through the continuous turnover of the medium thereby minimizing the effect of secretion and waste product accumulation. Second, the organ can be presented to different reagents without removal of the organ from the chamber. Third, the effects of nutrients and other chemical stimuli may be studied by collecting samples from the outlet of the chamber and analyzing such samples.

However, the perfusion, perifusion and superfusion systems of the prior art have several limitations. First, only a static concentration of a chemical stimuli can be supplied to the organ such that the concentration is known at every point in time. Varying the concentration dynamically by providing "pulses" of chemical stimuli results in unknown concentrations with respect to time of the chemical stimuli affecting the organ in the culture chamber. Consequently, research to determine the response of an organ to various concentrations of chemical stimuli using a dynamic system has been often times ineffective.

Second, since only a static or pulsed concentration of a nutrient or other chemical stimuli can be presented to the organ, overdose or underdose has been a frequent result. In trying to sustain organs in a biologically alive state, the supply of the proper concentration of nutrients to the organ as the organ's need for nutrients changes with respect to time is extremely important in extending the biological life of the organ or in attempting to analyze the response of an organ to a chemical stimuli in a dynamic situation.

Processes are also known for delivering a chemical to a biological tissue which use sensors to control delivery of the chemical and/or a feedback control scheme.

US—A—3 926 737 describes a process that controls variables such as gases that are dissolved into a culture medium on a real time basis through the use of sensors or rates measurement of the culturing vessel. The addition weights of the variables are calculated from the sensor value or the weight value.

US—A—4 064 015 discloses a process in which control is based on the measuring of the off-gases produced by the culture.

US—A—4 167 450 discloses a fermentation process wherein sensors are used to control delivery of the medium.

FR—A—2 293 488 describes a continuous method for the culture of microorganisms on a feedback-type basis, e.g. by measuring off-gases produced by the culture.

## Summary of the invention

It is an object of the present invention to provide a process and apparatus that supply to a biological tissue a chemical stimuli in a controlled manner, so that the concentration of the chemical stimuli affecting the tissue is known at each and every point in time allowing the user to emulate *in-vivo* conditions or other conditions.

The invention is characterized by the features of the claims.

The process includes starting with a first known volume of supportive media containing a known concentration of chemical stimuli affecting the tissue. Simultaneously, a first known volume of the supportive media containing a known amount of the chemical stimuli is removed from affecting the tissue thereby leaving a second known volume affecting the tissue; and a second known volume of the supportive media containing a known amount of the chemical stimuli is added to the second known volume affecting the tissue thereby forming a third known volume affecting the tissue. The second amount added has a different concentration which diffuses into the volume affecting the tissue, changing the concentration thereof. The concentration affecting the tissue is determined from knowing the previous volumes and their concentrations. The above steps are repeated such that the concentration of the chemical stimuli affecting the tissue is known at each and every point in time.

The process of the present invention controls the changing of the concentration of the chemical stimuli affecting the tissue with respect to time. When increases or decreases in concentration of the chemical stimuli are desired, the concentration of the chemical stimuli being added is increased or decreased. The process allows precise control of fluctuations of chemical stimuli with respect to time, whereby the amplitude, duration, rate dynamics, periodicity, frequency of oscillation, as well as notch, plateau, bimodal, and phase relationships can be studied.

Brief description of the drawings

Figure 1 is a graph illustrating the delivery of a static dose of a chemical stimuli or drug using a prior art process;

Figure 2 is a graph illustrating the delivery of a chemical stimuli or drug using a prior art process that changes the concentration of the chemical stimuli or drug in a step-type manner;

Figure 3 is a graph which illustrates a typical desired change in concentration of the chemical stimuli or drug that is to be delivered to a biological tissue;

Figure 4 is a graph which illustrates periods wherein the concentration of a chemical stimuli or drug is not known using prior art perifusion, perfusion and superfusion processes;

Figure 5 is a graph illustrating the ability of the present invention to control the concentration of two chemical stimuli or drugs simultaneously;

Figure 6 is a diagram illustrating the process of the present invention;

Figure 7 is a schematic view of the process of the present invention;

Figure 7a is a schematic view of an alternative embodiment of the present invention;

Figure 8 is a diagram of a suitable computer control system for controlling the process of the present invention;

Figure 9 is a graph illustrating the correlation between data points taken in an example in predicting the concentration of a drug in a culture chamber;

Figure 10 is a graph illustrating a plurality of possible R/V ratios;

Figures 11A and 11B are schematic diagrams of apparatus used in the example disclosed in the specification;

Figure 12 is a series of graphs showing the experimental results of the example disclosed in the specification;

Figure 13 is a graph illustrating the desired change in concentration with respect to time used in the example described in the specification;

Figures 14—16 are graphs which illustrate the estrogen secretion of the cultured ovaries in the example disclosed in the specification.

Detailed description of the preferred embodiments

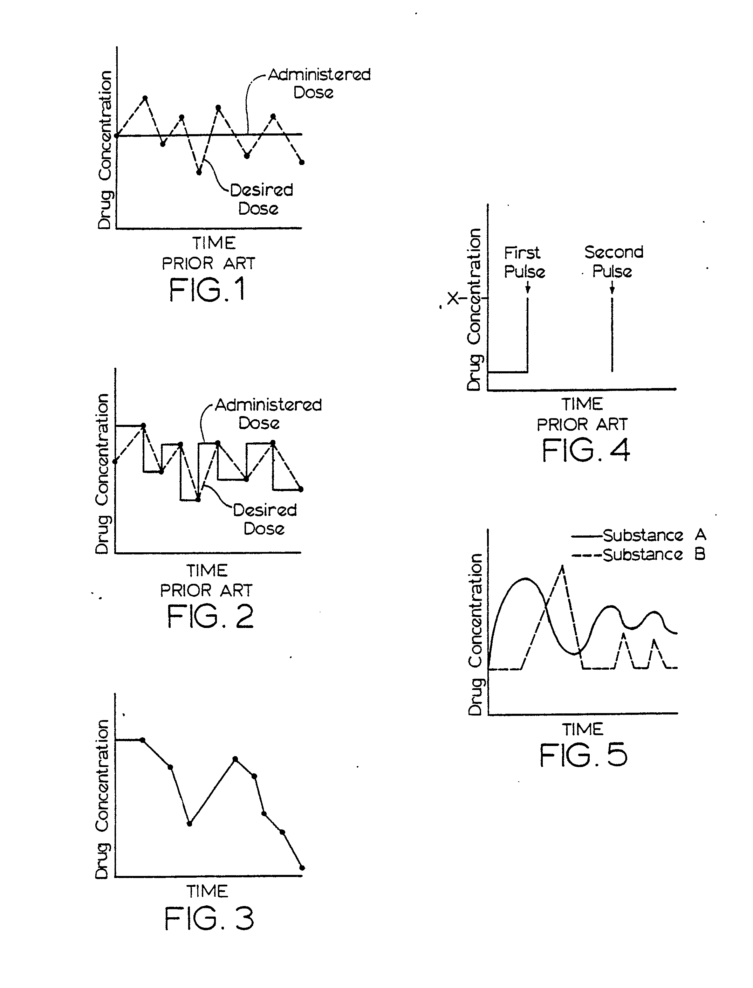

The process of the present invention emulates *in-vivo* conditions or other conditions to biologically alive tissue isolated from its natural nutrient supply. In the prior art, tissue that was isolated from its natural nutrient supply was either provided with a constant administered static dose of a chemical stimuli or drug, such as a nutrient or a hormone, as graphically illustrated in Figure 1 by a solid line, or the tissue was transferred to gradually increasing or decreasing chemical concentrations, resulting in a step type plot of concentration with respect to time as seen by the tissue, as illustrated in Figure 2 by a solid line. A desired pattern is indicated by a dashed line in each figure.

In a static system or steady dose process, illustrated in Figure 1, the tissue receives either an underdose or an overdose of the particular chemical stimuli or drug as time progresses which is quite unlike the manner in which the stimulus is naturally supplied in an *in-vivo* environment as most biological substance concentrations are dynamic with respect to time. The steady supply of a substance, such as a nutrient, in a constant concentration is typically used in a situation where an attempt is made to keep an organ alive for use as a transplant. Although a steady dosage will keep an organ alive for a period of time, the time period is quite short and the organ must be transplanted quickly. The static system makes no attempt to control the nutrient dosage delivered to the organ in a manner similar to the optimal manner in which the nutrient or drug would have been supplied *in-vivo*.

The other prior art process that increases or decreases concentrations is illustrated graphically in Figure 2. The tissue in this process is physically transferred to supportive media containing greater or lesser substance concentrations. The substance concentrations that the tissue is exposed to are consequently represented graphically in a step type fashion. Although the process that is graphically illustrated in Figure 2 more closely resembles *in-vivo* conditions than the static system described previously, underdosage and overdosage still occur, only in shorter time intervals. In addition, transferring of the organ from one concentration of substances to another is generally detrimental to the organ.

Other previous attempts, such as perifusion, perfusion and superfusion systems, to change the stimulant dosage being supplied to tissues to emulate *in-vivo* conditions have failed. Often times the substance dosage being supplied to the organ has to be changed quickly in a sequence of steps or several substances must change out of phase. For example, it may be desirable to decrease the drug dosage in two steps then increase in a third step and decrease again in four steps to respond to the needs of the organ as illustrated in Figure 3. With the organ being typically positioned within a culture chamber having an initial concentration of the substance and with a new and different concentration being fed into the culture chamber, by injecting a known concentration of the substance into the culture chamber in a known volume and allowing it to diffuse into the previous concentration in the culture chamber and reach equilibrium thus creating a new concentration. Since the initial concentration and volume of the substance affecting the organ is known and the concentration and volume of the substance injected is known, the new concentration affecting the organ at this time is known. However, since the system is a flow-through system, the concentration affecting the tissue will change in time. The result being the concentration of the substance affecting the organ becomes an unknown after the injection of the substance. This is graphically illustrated in Figure 4, wherein first and second pulses are illustrated and the concentration between the pulses is not known.

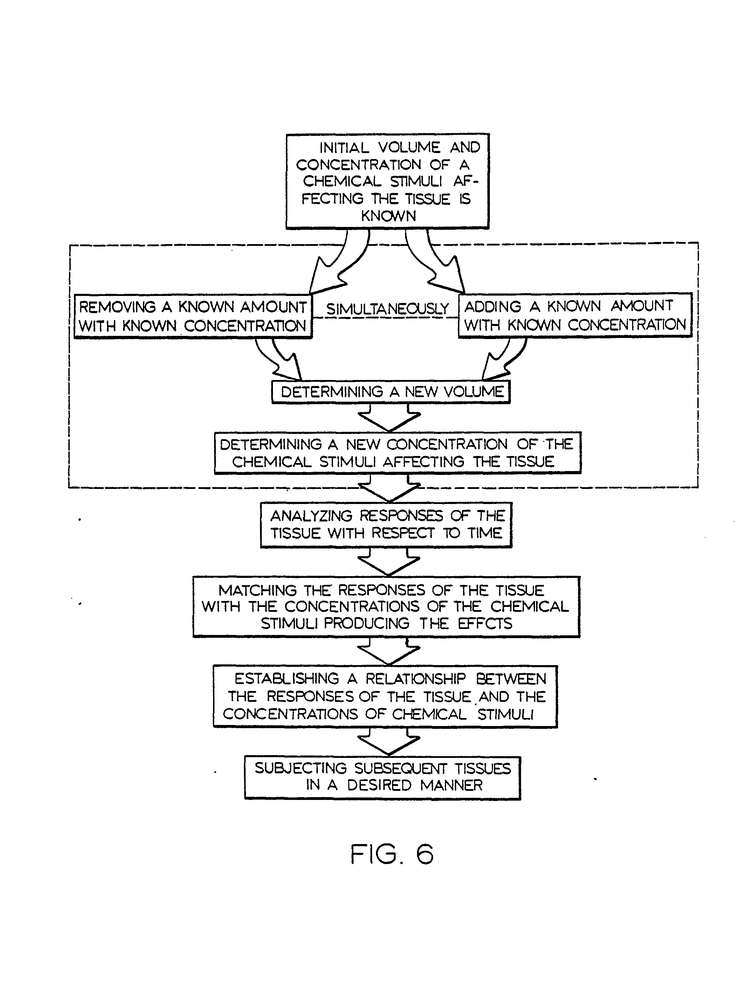

The process of the present invention can emulate the conditions of an *in-vivo* situation or any other conditions desired, as illustrated theoretically in a graph in Figure 5 of which none of the processes of the prior art can emulate exactly. In using the process of the present invention, the chemical stimuli dosages are changable in a controlled manner wherein the change in concentration affecting the tissue is substantially linear. The chemical stimuli concentration applied to the organ is known at any point in time and controllable at any point in time and a plurality of substances can be controlled in this manner simultaneously as indicated in Figure 6 by solid and dashed lines. The number of substances used depends on the solubility properties of the supporting media.

One important advantage of the process of the present invention is that the use of "intact organisms" (live animals) for experimentation can be avoided. Instead, tissue or organs are held *in-vitro*, under desired or in emulated *in-vivo* conditions. The effects of substances such as hormones, releasing factors, neurotransmitters, neuropeptide modulators, paracrines, "cybernins", growth factors, drugs, as well as glucose, tryptophan, lactate, and glycerol and other substances are determined by simulating known fluctuations of these substances and introducing them to isolated organs, tissue, or cell lines. The process provides an alternative to live animal testing, criticism of which is increasing every year, eliminating unnecessary pain and suffering of the animal and, reducing the cost of experimentation. The process emulates *in-vivo* conditions and allows tissues to respond in culture as they would in an intact organism.

For example, currently, Federal agencies, including the Consumer Product Safety Commission in the United States of America, require the use of the Draize test for cosmetic testing. An estimated 500,000 rabbits are utilized annually by cosmetic companies for this test which involves putting chemicals in living rabbits' eyes to identify agents that not only cause inflammation but also pain to the animals.

The Draize test involves the mechanism of an inflammatory response. Essentially, all tissues in mammalian organisms are capable of undergoing an inflammatory reaction in response to an irritant. The process of the present invention can maintain a select tissue (e.g., skin connective tissue, corneal cells) *in-vitro* and supply a pre-determined does of an irritant with respect to time while emulating normal *in-vivo* dynamics. Several mechanisms that indicate an inflammatory reaction are known and are easily assayed for. After determining the optimum conditions necessary to evoke the inflammatory response from known irritants *in-vitro*, the process can be used to simulate user application of the potential irritant. Monitoring the responses of tissues cultured in this manner provides a very accurate and inexpensive testing capability without pain and suffering inflicted on animals.

The process of the present invention is also applicable to pharmaceutical testing. Instead of injecting a "half-lethal" dose (LD-50) of a drug into live animals, the concentration of the drug over time is determined in the circulatory system of the animal and instead delivered to *in-vitro* isolated tissues under emulated *in-vivo* conditions. In this manner, the most optimum and beneficial modes of drug administration are resolved by determining the dose and time sequence of administration in a more physiologically effective manner. Side effects can be more readily determined and minimized and carcinogenic effects better accessed.

The pharmaceutical industry provides drug formulations or delivery systems that physicians use to command biological machinery. Systemic therapy consists of either pulses of drugs (e.g., at meal times), or of attempts to obtain constant plasma levels of a chemical. The viewpoint in either case is that there is a "therapeutic window"—a region of plasma concentration or total amount in the body below which the drug has little or no effect, and above which is toxic. This type of view may prove to be merely a primitive, technological approximation of what an optimally-patterned substance concentration should be, as there is more and more evidence that information is carried in waves of varying concentrations. These waves may be the signals that the cells process to determine their actions. The therapeutic window viewpoint has no dynamics and is thus not a signal. Utilizing the present process, medical scientists are provided with a means by which they can not only determine doses of drugs specified in amounts and schedule of administration, but also in rate of delivery. *In-vitro* testing in accordance with the present invention also allows side effects to be more readily determined and a direct cause of the drug accessed.

A further application includes the preservation of organ transplants. Typically, such organs can survive for very limited periods of time once they have been removed from their donors. Using a static drug dose system, an overdose or underdose of the nutrients needed to prolong the biological life of the organ occurs since the needs of the organ vary with respect to time as has been discussed previously. The process of the present invention is used initially to determine the optimal nutrient dosages needed to keep the organ biologically alive by supplying the nutrients mimicing *in-vivo* conditions and assaying the effluent for a biological response. Since the nutrient dosage at each and every point in time affecting the organ is known, these nutrient dosages can be matched to the assay results to determine a predetermined program of administering a desired dosage at a desired time. Once a schedule for predetermined administration has been determined, the process of the present invention is then used to supply the desired concentrations at the desired times to the organ, allowing the organ to respond to a series of chemical stimuli as it would *in-vivo* for periods of time never before achieved.

Using the present process, it has been demonstrated that rat ovaries can be maintained and remain physiologically viable during a simulated four day cycle *in-vitro*. They responded to a series of chemical stimuli which mimiced the *in-vivo* condition of the rat by ovulating at the end of the four day cycle. In addition, the ovaries grew and secreted substances very similar to ovaries remaining *in-vivo*. Those skilled in the art will recognize this as a remarkable accomplishment. There was no indication that the ovaries would not continue to respond in this manner for extended periods of time.

Still a further application includes intravenous administration of drugs or the like. The dosage of a drug or the like being delivered to a patient is controllable over time. Consequently, once a predetermined administration of the drug has been determined, the drug is deliverable at desired dosages at desired times.

Since it is known that all biological phenomena are physiochemical, there will always be an appropriate dynamic description. Utilizing the present process it is now possible to study the effects of these dynamics to discover how they control cellular activities. The goal is ultimately to control the response of cells for therapeutic purposes. With this achieved, the information would be invaluable in finding a cure for cancer, diabetes, birth defects, aging complications, obesity and diseases involving hormonal imbalances to name just a few.

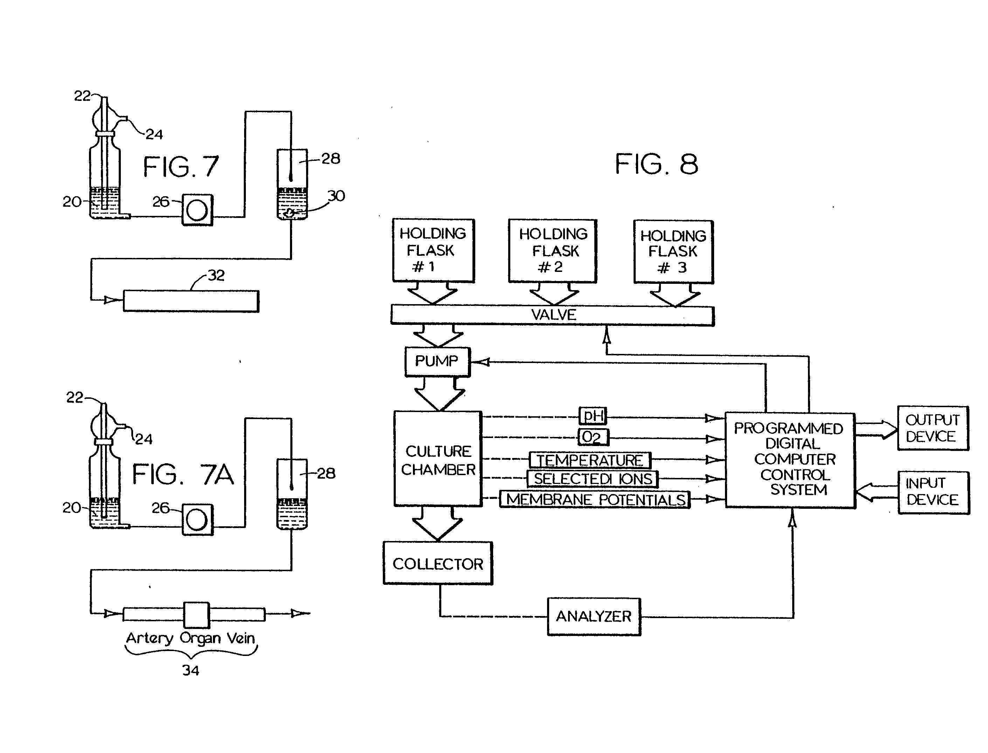

The process of the present invention is diagrammatically illustrated in Figure 6. Typically, the tissue is removed from its natural environment and nutrient supply and placed in a system schematically illustrated in Figure 7. The system illustrated in Figure 7 includes a holding flask 20 having a gas inlet 22 and pressure release 24. A pump 26 pumps the chemical stimuli or drug into a culture chamber 28 wherein the tissue 30 is positioned. An initial known volume with a known concentration of a chemical stimuli (drug or nutrient) is supplied to the tissue 30 in the culture chamber 28. A collection system 32 collects perfusate for analysis.

Alternatively, the tissue is left in the body but severed from its natural nutrient supply. The chemical stimuli, such as a nutrient, is supplied to the tissue through its natural cardiovascular system 34 similar to a perfusion system, as illustrated in Figure 7a.

A known amount of the chemical stimuli affecting the tissue is then removed from the first known volume in a second known volume. Consequently, the remaining volume and its chemical stimuli concentration are known. Simultaneously, a known amount with a known but different concentration of the chemical is added to the volume affecting the tissue in a third known volume. Consequently, the final volume affecting the tissue along with the final concentration is determinable.

The immediately above-mentioned steps are repeated, and if done on a continual basis, define a continuous flow system applicable to both a culture chamber perifusion system or a perfusion system.

In order to alter the concentration of substances in the culture chamber, a plurality of holding flasks is preferably used, each containing different concentrations of substances than the starting concentration of the substances in the culture chamber. For example, if the concentration of the substance within the holding flask had a higher value than the substance in the culture chamber, the concentration in the culture chamber would increase over time as the supply substance from a particular holding flask replaced the substance in the culture chamber.

The change in concentration of the substance within the culture chamber in response to a known supply substance concentration being delivered to the culture chamber with a known volume at a known rate can be readily determined by use of the system in conjunction with a digital computer.

In the present process a certain rate dependent volume was removed from the culture chamber during a known time interval. Simultaneously, a drop of another rate dependent volume is being formed at the top of the culture chamber. Eventually, this drop enters the culture chamber and equilibrates with the substance in the culture chamber.

To initiate the simulation sequence, the following parameters were defined:

Culture Chamber Volume (V)—ml

Culture Chamber Starting Concentration $(C_i)$—units/ml

Supply Drug Starting Concentration in Holding Flasks $(C_s)$—units/ml

Rate of Fluid Delivery (R)—ml/hr

Integration Interval (T)—seconds

With these parameters defined, the following loops were programmed in the digital computer to simulate the process. The following jobs were performed during each loop for each chemical stimuli:

1. The total number of units $(U_T)$ in the culture chamber was determined $(U_T=C_i$ units/ml$\times V$ ml).

2. A volume $(V_R)$ was removed from the culture chamber. $V_R$ is a function of R and T

$$V_R=\frac{R\,ml/hr\times T\,sec}{3600\,hr/sec}.$$

3. The new volume of the culture chamber was determined $(V=V-V_R)$.

4. The amount of units removed $(U_R)$ from the culture chamber with $V_R$ was determined. $U_R$ is a function of $C_i$ and $V_R$ $(U_R=(C_i$ units/ml$)$ $(V_R$ ml$))$.

5. The new $U_T$ in the culture chamber was determined $(U_T=U_T-U_R)$.

6. A volume was added to the culture chamber $(V_A)$ from the holding flask. If the culture chamber was airtight, $V_A$ would be equivalent to $V_R$ $(V_A=V_R)$.

7. The new volume of the culture chamber was determined $(V=V+V_A)$.

8. The amount of units added to the culture chamber $(V_A)$ with $V_A$ was determined $(V_A=(C_i$ units/ml$)$ $(V_A$ ml$))$.

9. The new $U_T$ in the culture chamber was determined $(U_T=U_T+U_A)$.

10. The new $C_i$ was determined

$$C_i=\frac{U\,units}{V\,ml}$$

When the culture chamber volume is kept constant, the concentration of any diffusable substance can be determined mathematically (equation (1)). This equation is derived from the computer-aided analysis of the process.

$$C(t)=C_s+(C_i-C_s)e^{-RT/V}. \qquad (1)$$

$C_{(t)}$=concentration in culture chamber at time T

$C_s$=concentration of chemical stimulus in holding flask (final culture chamber concentration at T=$\infty$)

$C_i$=initial culture chamber concentration (t=0)

R=rate of supply substance delivery from holding flask to culture chamber (ml/hr)

V=volume of media in culture chamber (ml)

T=time (hrs)

To test the ability of equation (1) to accurately predict the concentration of the substance in the culture chamber when either a higher or lower concentration of the same drug was being delivered to the culture chamber at a constant rate, the following experiments were conducted. A dye, methyl orange (MO), was selected as the supply substance in these experiments because: 1) the concentration of the dye in the culture chamber could be easily assayed by measuring percent transmission at a maximum absorbance of 460 nanometers utilizing a spectrophotometer, and 2) at the completion of the assay, the sample of culture chamber fluid could be placed back into the culture chamber. This is important because it allowed continuous sampling over time without altering any of the parameters in equation (1) (i.e., culture chamber volume and $C_i$).

To test the ability of equation (1) to predict changes in culture chamber concentration when a higher concentration of the same drug was delivered at a constant rate from the holding flask, 100 ml of a standardized solution was placed in the holding flask. This solution of MO was adjusted so that it produced a reading of 10% transmission and was considered to be a 100% concentrated solution. 20 ml of distilled water (100% transmission) was placed in the culture chamber and was considered a 0% concentrated solution. The values between 10 and 100 percent transmission lie on the linear portion of a standard curve with a correlation coefficient of 0.99995 comparing known concentrations of MO and percent transmission. The 100% solution was pumped into the culture chamber at a rate of 18.5 ml/hr. At 15 minute intervals over a period of two hours, the pump was turned off and a 2 ml sample from the culture chamber was removed and assayed with a photospectrometer. The percent transmission was converted to optical density and the concentration recorded from the standard curve. The sample was then replaced in the culture chamber and the pump restarted.

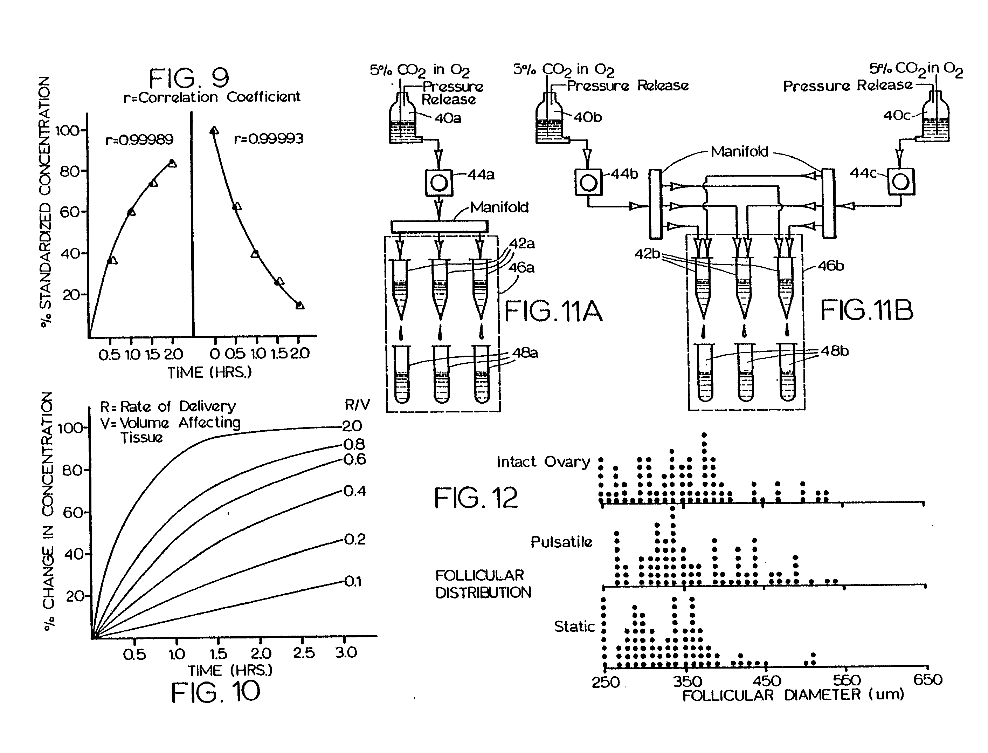

To test the ability of equation (1) to predict changes in culture chamber concentration when a lower concentration of the supply drug was delivered from the holding flask, 100 ml of distilled water (0% concentration) was placed in the holding flask. 20 ml of a 100% concentrated solution of MO was placed in the culture chamber. The 0% concentrated solution was then pumped into the culture chamber and the methyl orange concentration determined as described above. The results of these experiments are shown in Figure 9. They clearly indicate that equation (1) can be used as an expression to describe the present process.

With the capability of being able to accurately predict concentration changes in the culture chamber, it is now possible to emulate any data where a diffusable substance concentration is plotted as a function of time, in the culture chamber. With the initial concentration of the substance in the culture chamber known and the value of the desired final concentration in the culture chamber over a known time interval also known ($C_s$ . $_t$). The concentration of the substance to be placed in the holding flask can be determined by a manipulation of equation (1) to derive equation (6).

$$C_{(t)}=C_s+(C_i-C_s)e^{-RT/V} \qquad (1)$$

since $C_s$ . $_t=C_{(t)}$, substituting

$$C_s . _t=C_s+(C_i-C_s)e^{-RT/V} \qquad (2)$$

factoring:

$$C_s . _t=C_s+C_ie^{-RT/V}-C_se^{-RT/V} \qquad (3)$$

subtracting $C_ie^{-RT/V}$ from each side:

$$C_s . _t-C_ie^{-RT/V}=C_s-C_se^{-RT/V} \qquad (4)$$

factoring:

$$C_s . _t-C_ie^{-RT/V}=C_s(1-e)^{-RT/V} \qquad (5)$$

solving for $C_s$:

$$C_s=\frac{C_s . _t-C_ie^{-RT/V}}{1-e^{-RT/V}} \qquad (6)$$

To more precisely emulate *in-vivo* conditions, a desired rate of delivery (R) and a desired volume (V) affecting the tissue in the culture chamber must be determined. In other words, if it is desirous to change the concentration of the substance within the culture chamber from the initial concentration ($C_i$) to a different concentration (C(t)) linearly with respect to time, the change from $C_i$ to C(t) is a function of R and V. As shown graphically in Figure 10, a ratio of R/V affects the maximum percent change that can occur over a known time interval. The maximum percent change that is possible during a known time interval varies directly with the R/V ratio. However, the linearity of the line between the initial point ($C_i$) and the final point (C(t)) over a known time interval varies inversely to the R/V ratio.

If a large linear increase in concentration in the culture chamber is desired during a given time interval, the slope of a line from $C_i$ to C(t) is also great. Since the linearity of the increase in concentration in the culture chamber is an inverse function of the R/V ratio, large increases pose problems in reaching the point C(t) in a given time interval in a linear fashion. The linearity of the change in concentration can be preserved by increasing the concentration of the substance being supplied, thus not affecting the R/V ratio.

This is not the case if a large decrease in concentration in the culture chamber is desired, as the minimum concentration of a substance in a holding flask can be zero. Thus, the minimum R/V ratio (maximum linearity) must be determined. This R/V ratio is then compared with Figure 10. To determine if the calculated R/V ratio will emulate the desired degree of linearity between the data points ($C_i$ and C(t)) in question.

To determine the minimum R/V ratio required to achieve the percent concentration change between C and C(t), the negative slope for the decreasing concentration is analyzed over a known desired time interval wherein the supply substance concentration ($C_s$) equals zero. The amount of change ($\Delta_c$) between $C_i$ and C(t) over the time interval t is determined by equation (7):

$$\Delta_c=\frac{C_i-C(t)}{C_i} \qquad (7)$$

A 100% change would occur if C(t) was zero ($\Delta_c=1$). Thus, the concentration at C(t) would be the difference between a 100% change ($\Delta_c=1$) and the actual change, as expressed by equation (8):

$$C_{(t)}=1-\Delta_c \qquad (8)$$

To drive to a 100% change the supply substance concentration ($C_s$) must be zero. If the initial concentration ($C_i$) is assumed to be one, equation (8) can be substituted in equation (3), the determination of which is discussed subsequently, with $C_s=0$ and $C_i=1$ and an expression for the minimum R/V ratio derived:

$$C_{(t)}=C_s+(C_i-C_s)e^{-RT/V} \qquad (9)$$

substituting equation (2) into (3) to obtain equation (4):

$$1-\Delta_c=0+(1-0)e^{-RT/V} \qquad (10)$$

simplifying:

$$1-\Delta_c=e^{-RT/V} \qquad (11)$$

simplifying:

$$1n(1-\Delta_c)=^{-RT/V} \qquad (12)$$

solving for R/V:

$$\frac{R}{V}=\frac{-1}{T}1n(1-\Delta_c) \qquad (13)$$

substituting equation (7) into equation (13):

$$\frac{R}{V}=\frac{-1}{T}1n(1-\frac{C_i-C_{(t)}}{C_i}) \qquad (14)$$

simplifying:

$$\frac{R}{V} = \frac{-1}{T}\ln\left(\frac{C_{(t)}}{C_i}\right) \qquad (15)$$

Equation (15) provides an expression for the minimum R/V ratio required to drive to a desired concentration in a given time interval in the culture chamber. If the linearity provided by the calculated R/V ratio is not adequate to emulate a given set of data, the change in concentration can be broken into at least two steps. Assuming we wish to emulate a set of data that changes linearly from an initial concentration of $C_i$ to a final concentration of C(t) in a time interval of t, the first step is to break this change into two intervals. This is accomplished by setting a new point (A) at a point half way between $C_i$ and C(t) (i.e., at t=1/2t). Thus, secondly, a new $C_s$ in the holding flask and a new minimum R/V ratio can be calculated using $C_i$ as the initial point and changing point A to a new C(t) for a final point. Since the percent change required between $C_i$ and A is less than between $C_i$ and the original C(t), the minimum R/V will be lower and the linearity increased. This procedure can be repeated until a satisfactory linearity is achieved. In this manner, the process of the present invention emulates *in-vivo* conditions and other conditions and provides a method of knowing the concentration affecting the tissue at each and every point in time.

In practice, the supply substance concentration is changed using a preferred method wherein a plurality of holding flasks is used with each flask containing a different concentration ($C_s$) of the supply substance. When a different concentration of the supply drug is needed, a change is made from one holding flask to another. It will be understood by those skilled in the art that the number of flasks needed and the differences between the concentrations of the supply drug in the flasks will be a function of the particular application of the process.

Peristaltic pumps are a preferred apparatus for conveying the supply substance from the holding flasks to the culture chamber. The rate of delivery is controlled by a suitable variable speed motor and controller which drive the pump.

The known amounts removed from the culture chamber are then assayed to determine the response of the tissue to the substance. In addition, the culture chamber may be designed to accept probes, which are attached to suitable instrumentation, for monitoring pH, oxygen consumption, temperature, selective ions, membrane potentials and other variables of interest.

With the responses of the tissue known as a function of time and with the concentrations of the substance affecting the tissue also known as a function of time, relationships are established as to cause and effect, through known mathematical methods. With the immediately above-described procedure repeated and performed against "control" specimens, proper drug concentration and administration to a particular tissue are determined.

As will be appreciated by those skilled in the art, the present process is applicable to the delivery of more than one chemical stimuli at a time to the culture chamber by mixing the substances together in the same holding flask. In the case of multiple substances being utilized, the minimum R/V ratio must be determined from the greatest slope in each time interval. This result will be the same for all changes in that interval. Complex experiments, heretofore not possible, may be carried out *in-vitro* emulating known *in-vivo* and other conditions by use of the present invention. *In-vitro* conditions are adjustable at will and the responses observable without costly live animal experimentation. In addition, unwanted interference from bodily responses to experimentation directed at the particular tissue is eliminated thereby reducing the number of experiments needed to achieve statistical reliability.

Once the relationships between chemical stimuli and tissue are determined, the process is used to deliver the chemical stimuli to subsequent tissue in an optimal manner. For example, organ transplants are kept biologically alive longer using the process to deliver nutrients at a rate and concentration as the transplant needs the nutrients.

Another important advantage of the process of the present invention is that it is particularly well suited for control by electronic means such as a programmed digital computer together with appropriate interface circuitry, as illustrated in Figure 8. More specifically, the administration of the rate of delivery and the concentrations of the chemical stimuli supplied to the tissue are controllable by a suitable microprocessor-based control system. Once programmed with the desired changes in concentration with respect to time, the microprocessor manipulates the process apparatus, such as the peristaltic pump. If a plurality of flasks are used to hold different concentrations of the chemical stimuli, then the switching from flask to flask is controlled through the microprocessor.

To more fully explain the process of the present invention, an example is set forth below. The example is set forth in particular detail and should not be construed as limiting the scope of the process of the present invention in any regard.

Example
Introduction

This experiment is designed to test whether a hormone administered in a wave form manner elicits a different response from an isolated organ than the same amount of hormone administered in a static matter.

The model system utilized is the isolated metestrous rat ovary. Luteinizing hormone (LH) is known to be released from the anterior pituitary of the brain in a pulsatile manner in the rat. This results in the plasma concentration of LH to fluctuate in the rat body such that hourly peaks in LH concentration occur. These peaks are illustrated in Figure 11. Before the process of the present invention, the significance of these fluctuations could not be tested.

LH is known to stimulate the production of androgen. This indirectly affects the rate of growth of the follicles within the ovary. Therefore to test whether LH has different effects when administered in a pulsatile versus a static manner, the amount of estradiol-17β secreted and the follicular growth patterns were assayed. The hormone FSH activates an enzyme to convert androgen to estrogen such as estradiol-β.

Animals

Nine mature female Sprague-Dawley rats were kept under controlled conditions of temperature, humidity and light. The animals were exposed to a 12 light/12 dark cycle. Estrous cycles were monitored by taking daily vaginal smears between 0800 hours and 1000 hours. The mature rats were observed for three consecutive cycles before being placed on experiment. Only rats without signs of respiratory distress, mammary tumors, or other gross pathology were used in this experiment.

Culture system

A schematic diagram of the continuous flow system used in this study is illustrated in Figure 11. 500 ml glass aspirating flasks were used as holding flasks 40a—c. 20 cc syringes were used as culture chambers 42a, 42b. The media within the holding flasks 40a—c was delivered to the culture chambers 42a, 42b at a constant rate of 22.8 ml/hr by peristaltic pumps 44a—c through Tygon food tubing (3.175 mm (1/8″) O.D.). The culture chambers 42a, 42b were kept airtight by a rubber seal, thus the volume of media in the culture chambers 42a, 42b remained constant. The culture chambers 42a, 42b were maintained in a Thelco incubator at 37°C. The perfusate was collected from each culture chamber 42a, 42b by a fraction collector 48a, 48b. The fractions were collected every 15 minutes.

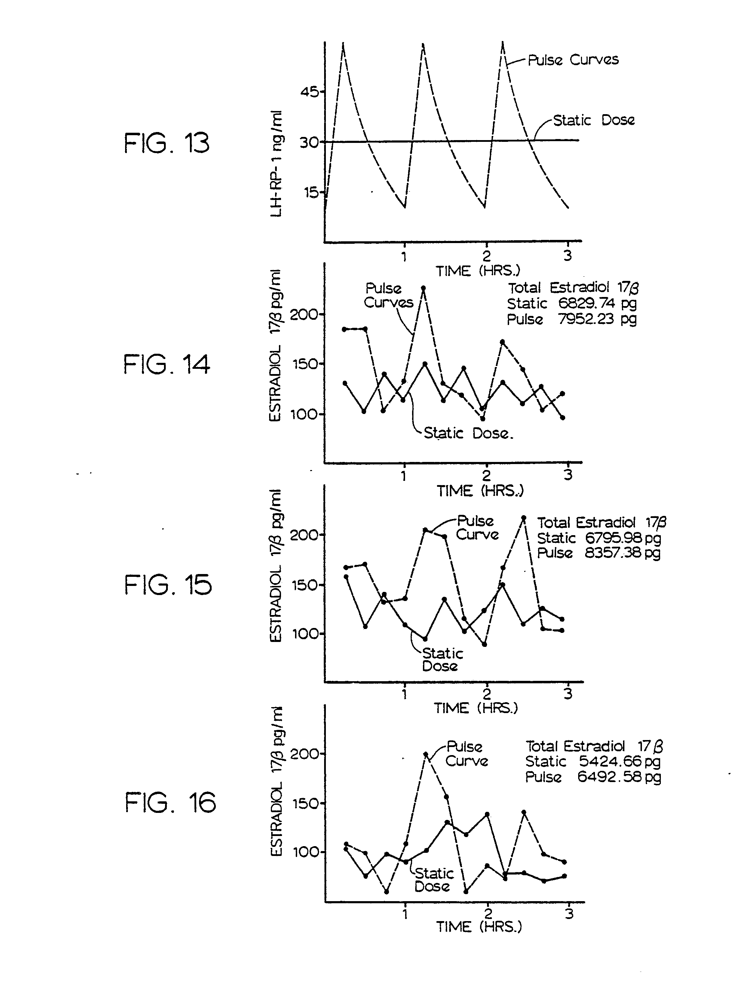

In order to mimic the three hourly pulses of LH illustrated in Figure 13, two holding flasks were utilized per culture chamber. The first holding flask 40b contained 144 ng/ml of LH-RP-1 and 200 ng/ml of FSH-RP-1. The holding flask 40c contained 0.56 ng/ml LH-RP-1 and 200 ng/ml FSH-RP-1. The culture chamber 42b had a starting concentration of 10 ng/ml LH-RP-1 and 200 ng/ml FSH-RP-1. The first holding flask 40b was delivered to the culture chambers for the first 0.2 h of each hour of the experiment. After 0.2 h a first holding flask pump 44b was shut off and the second holding flask pump 44c was started and run for the remaining 0.8 h of the hour. In this manner, the graph shown in Figure 14 could be precisely emulated in the culture chamber 42b.

To provide a control to test the effects of LH administered in a pulsatile manner, the total amount of LH that would be delivered in the three hour period was determined by integrating the area under the pulse curves in Figure 13. The results of the integration was used to determine a static dose of LH to be administered for the three hour interval that would provide exactly the same amount of LH to the culture chambers during the three hour interval. 29.25 ng/ml LH-RP-1 was calculated to provide a control and was mixed in the holding flask 40a with 200 ng/ml FSH-RP-1. This mixture was delivered to the culture chamber 46a with the same starting concentrations.

Preparation of reagents

Bovine luteinizing hormone (NIAMDD-bLH-4) with a biopotency of 2.4 units/mg (one unit of activity=1.0 mg of NIH-LH-S1) and ovine follicle stimulating hormone (NIAMDD-oFSH-14) with a biopotency of 9.0 units/mg (one unit of activity=1.0 mg of NIH-FSH-S1) were prepared by serial dilutions of 1.0 mg of hormone in 10 ml of physiological saline. Final stock solutions of 500 ng/ml LH and 50,000 ng/ml FSH were prepared in supplemented medium 199.

Medium 199 (Gibco) was prepared by a 10-fold dilution of a concentrated solution using double distilled deionized water. The solution was supplemented with 2.2 g/L $NaHCO_3$, 2 g/L Bovine Serum Albumen, 1 g/L glucose, 50 mg/L streptomycin sulfate, and 100,000 I.U./L K-penicillin-G (stock TC 199). The media was dispensed by a repippetting system (Labindustries) accurate to ±0.10 ml to the holding flasks and the culture chamber. Microliter volumes of the stock hormone solution were added with micropipettes (Oxford) with an accuracy of ±0.05 ml. All media was subsequently equilibrated with 5% $CO_2$ in oxygen and the pH adjusted to 7.40±0.05. The media was then passed through a millipore filter (HA45), transferred to sterile holding flasks and culture chamber and stored at 5°C. All holding flasks were warmed to 25°C (culture chamber was warmed to 37°C), injected with 200 I.U./L regular insulin (Lente) and equilibrated with 5% $CO_2$ in oxygen 20 minutes prior to use. The holding flasks were gassed for 20 sec/10 minutes at a pressure of less than 7 kPa (1 psi) while in operation.

Experimental protocol

Three mature rats were autopsied between 0930 h and 0950 h on the morning of metestrus. The ovaries were excised, trimmed of excess fat, and placed in stock TC-199 warmed to 37°C supplemented with 200 I.U./L insulin (I) and 400 I.U./L heparin sulfate (H) which was previously equilibrated with 5% $CO_2$ in oxygen. After 10 minutes, the ovaries were loaded into the nine culture chambers. One ovary from each animal went into a "pulsatile" chamber and the other into a "static" chamber. At 1000 h the peristaltic pumps were started and all chambers were exposed to the appropriate hormone levels.

Results

Figure 12 shows the results of the follicular distribution patterns. The top tier shows the follicular distribution pattern of three uncultured ovaries (intact ovaries). The middle tier shows the distribution pattern from the cultured ovaries exposed to pulsatile LH stimulation of the present invention. The pattern was not significantly different than the intact ovaries, but was significantly (Probability<.05 using chi square test) different than the cultured ovaries exposed to static LH stimulation shown in the bottom tier.

Figures 14—16 illustrate the estrogen secretion of the cultured ovaries from each animal. The solid lines, in Figures 14—16, indicate the ovary exposed to static LH stimulation and the dotted lines indicate the ovary exposed to pulsatile LH using the process of the present invention. Both static and pulsatilely stimulated ovaries exhibited a pulsatile release of estrogen. However, the pulsed ovaries exhibited three distinct peaks of estrogen secretion which correlated with the LH pulses. In addition, significantly more estrogen was secreted from the pulsatilely stimulated ovaries (Probability<0.01 using two-tailed students "t" test) than the statically stimulated ovaries.

Conclusions

The process of the present invention is a powerful research tool and allows the study of the dynamics of internal blood signals. These results provide evidence that it is not only the amount of hormone that controls cellular function, but also the manner in which the hormone is delivered.

**Claims**

1. A process for delivering a chemical in a predetermined concentration pattern to a biological tissue wherein the chemical concentration is changed over a total time period, the processing comprising:

(a) selecting a pattern of changing concentration of the chemical extending and changing over the total time period;

(b) splitting the total time period into a plurality of chronologically arranged smaller time periods, each smaller time period being free from any inflection point in the pattern of changing concentration, and having an initial concentration level and a final concentration level of the chemical;

(c) selecting a rate of delivery of the chemical and a concentration of the chemical being delivered in the smaller time period according to a concentration curve being defined by

$$C_{(t)}=C_s+(C_i-C_s)e^{-Rt/V}$$

wherein

t=the smaller time period

V=volume of fluid within a chamber affecting the cell or tissue in which the chemical is being delivered

R=the rate of delivery of the chemical to the chamber

$C_i$=the initial concentration level of the chemical in the smaller time period within the chamber

$C_{(t)}$=the final concentration of the chemical in the smaller time period within the chamber

$C_s$=the concentration of the chemical being delivered in the smaller time period from a supply stream into the chamber

and which is approximately linear and includes $C_i$ and $C_{(t)}$ by selecting values of R/V and $C_s$;

(d) providing a supply source containing the chemical with a concentration greater than $C_s$ for each smaller time period;

(e) delivering the chemical from the supply source to the chamber at the rate R and the concentration $C_s$ as defined in (c);

(f) repeating (c) through (e) for each subsequent smaller time period such that the pattern selected is substantially emulated to obtain the response of the cell or tissue to the delivery of the chemical according to the pattern of changing concentration.

2. The process of claim 1 wherein the cell or tissue is in a culture chamber and wherein the chamber that the concentration level $C_s$ is being delivered to is in fluid communication with the culture chamber.

3. The process of claim 1 wherein the chamber is a culture chamber containing the tissue.

4. The process of claim 1 wherein the tissue is an organ and delivering the chemical from the chamber to the organ.

5. The process of claim 1 and further including the step of:

performing steps (b) and (c) using a programmed digital computer.

6. The process of claim 1 and further including:

delivering the chemical at a constant concentration to a similar cell or tissue in another chamber for comparison with the response of the cell or tissue to the delivery of the chemical according to claim 1.

7. The process of claim 1 wherein the biological tissue is a cell.

8. The process of claim 1 wherein the tissue is isolated by being positioned in a culture chamber having an inlet through which the chemical is supplied and an outlet through which the chemical is removed.

9. The process of claim 1 wherein a plurality of chemicals are delivered to the biological tissue, each in its own predetermined concentration pattern, according to the process described in claim 1.

10. An apparatus for delivering a chemical in a predetermined concentration pattern to a biological tissue with a process according to any of claims 1 to 9, starting with a first known volume of the chemical and having a known concentration affecting the tissue, the apparatus comprising:

a) means for removing a first known amount of the chemical from affecting the tissue thereby leaving a second known volume having a second known concentration affecting the tissue;

b) means for adding a second known amount of the chemical having a different concentration to the second known volume affecting the tissue thereby forming a third known volume affecting the tissue;

c) means for determining a third known concentration of the third known volume of the chemical affecting the tissue based upon the removal of the first known amount and the addition of the second known amount; and

d) means for controlling operation of the means for adding, the means for removing, and the means for determining such that the change in concentration of the chemical affecting the tissue is changing in a substantially linear manner and is known at each and every point in time.

11. The apparatus of claim 10 wherein the means for controlling operation of the means for adding, the means for removing, and the means for determining is a programmed digital computer.

12. The apparatus of claim 10 and further including:

means for analyzing selected first known amounts of the chemical removed from the first volume affecting the tissue;

means for matching the selected analyzed first amounts of the chemical with chronologically corresponding third known concentrations of the chemical affecting the tissue for establishing a relationship of tissue response with respect to selected concentrations of the chemical; and

means for combining the individual responses and the corresponding concentrations of the chemical to form a relationship establishing the reaction of the tissue to change in concentration of the chemical over time.

**Patentansprüche**

1. Verfahren zum Liefern eines chemischen Stoffes in einem bestimmten Konzentrationsmuster an ein biologisches Gewebe, bei dem die Konzentration des chemischen Stoffes über einen gesamten Zeitraum geändert wird, mit den folgenden Verfahrensschritten:

(a) Wählen eines Musters für die Änderung der Konzentration des chemischen Stoffes, das sich über den gesamten Zeitraum erstreckt und ändert;

(b) Aufteilen des gesamten Zeitraumes in mehrere chronologisch angeordnete kürzere Zeiträume, wobei jeder kürzere Zeitraum keinen Wendepunkt in dem Muster der sich ändernden Konzentration aufweist und ein ursprüngliches Konzentrationsniveau und ein abschließendes Konzentrationsniveau des chemischen Stoffes hat;

(c) Wählen einer Liefergeschwindigkeit des chemischen Stoffes und einer Konzentration des in dem kürzeren Zeitraum gelieferten chemischen Stoffes gemäß einer Konzentrationskurve, die definiert ist durch

$$C_{(t)}=C_s+(C_i-C_s)e^{-Rt/V}$$

wobei

t=der kürzere Zeitraum

V=Fluidvolumen innerhalb einer Kammer, das die Zelle oder das Gewebe beeinflusst, an das der chemische Stoff geliefert wird

R=die Liefergeschwindigkeit des chemischen Stoffes an die Kammer

$C_i$=das ursprüngliche Konzentrationsniveau des chemischen Stoffes in dem kürzeren Zeitraum innerhalb der Kammer

$C_{(t)}$=die abschließende Konzentration des chemischen Stoffes in dem kürzeren Zeitraum innerhalb der Kammer

$C_s$=die Konzentration des chemischen Stoffes, der in dem kürzeren Zeitraum von einem Versorgungsstrom in die Kammer geliefert wird

und die annähernd linear ist und $C_i$ und $C_{(t)}$ umfaßt durch Wahl von Werten für R/V und $C_s$;

(d) Vorsehen einer Versorgungsquelle, die den chemischen Stoff für jeden kürzeren Zeitraum mit einer Konzentration größer als $C_s$ enthält;

(e) Liefern des chemischen Stoffes von der Versorgungsquelle zu der Kammer mit der unter (c) definierten Geschwindigkeit R und der Konzentration $C_s$;

(f) Wiederholen der Schritte (c) bis (e) für jeden aufeinanderfolgenden kürzeren Zeitraum, so daß das gewählte Muster im wesentlichen nachgeahmt wird, um die Antwort der Zelle oder des Gewebes auf die Zufuhr des chemischen Stoffes gemäß dem Muster der sich ändernden Konzentration zu erhalten.

2. Verfahren nach Anspruch 1, dadurch gekennzeichnet, daß die Zelle oder das Gewebe sich in einer Kulturkammer befindet und daß die Kammer, der das Konzentrations niveau $C_s$ zugeführt wird, in Fluidverbindung mit der Kulturkammer steht.

3. Verfahren nach Anspruch 1, dadurch gekennzeichnet, daß die Kammer eine Kulturkammer ist, die das Gewebe enthält.

4. Verfahren nach Anspruch 1, dadurch gekennzeichnet, daß das Gewebe ein Organ ist und der chemische Stoff von der Kammer dem Organ zugeführt wird.

5. Verfahren nach Anspruch 1, ferner dadurch gekennzeichnet, daß die Schritte (b) und (c) unter Verwendung eines programmierten Digitalrechners durchgeführt werden.

6. Verfahren nach Anspruch 1, ferner dadurch gekennzeichnet, daß der chemische Stoff einer ähnlichen Zelle oder einem ähnlichen Gewebe in einer anderen Kammer mit konstanter Konzentration zugeführt wird zum Vergleich mit der Antwort der Zelle oder des Gewebes auf die Zufuhr des chemischen Stoffes gemäß Anspruch 1.

7. Verfahren nach Anspruch 1, dadurch gekennzeichnet, daß das biologische Gewebe eine Zelle ist.

8. Verfahren nach Anspruch 1, dadurch gekennzeichnet, daß das Gewebe dadurch isoliert wird, daß es in eine Kulturkammer gebracht wird, die einen Einlaß aufweist, durch den der chemische Stoff zugeführt wird, und einen Auslaß, durch den der chemische Stoff entfernt wird.

9. Verfahren nach Anspruch 1, dadurch gekennzeichnet, daß im biologischen Gewebe mehrere chemische Stoffe jeweils gemäß ihrem eigenen, bestimmten Konzentrationsmuster gemäß dem Verfahren nach Anspruch 1 zugeführt werden.

10. Vorrichtung zum Liefern eines chemischen Stoffes in einem bestimmten Konzentrationsmuster an ein biologisches Gewebe mit einem Verfahren nach einem der Ansprüche 1—9, ausgehend von einem ersten bekannten Volumen des chemischen Stoffes, der eine bekannte, das Gewebe beeinflussende Konzentration aufweist, mit:

a) einer Einrichtung zum Entfernen einer ersten bekannten Menge des chemischen Stoffes, so daß diese das Gewebe nicht mehr beeinflußt, wobei ein zweites bekanntes Volumen mit einer zweiten bekannten Konzentration zurückgelassen wird und das Gewebe beeinflußt;

b) einer Einrichtung zum Addieren einer zweite bekannten Menge des chemischen Stoffes mit einer unterschiedlichen Konzentration zu dem zweiten bekannten Volumen, das das Gewebe beeinflußt, wobei ein drittes bekanntes Volumen gebildet wird, das das Gewebe beeinflußt;

c) einer Einrichtung zum Bestimmen einer dritten bekannten Konzentration des dritten bekannten Volumens des das Gewebe beeinflussenden chemischen Stoffes aufgrund des Entfernens der ersten bekannten Menge und des Addierens der zweiten bekannten Menge; und

d) einer Einrichtung zum Steuern des Betriebs der Addiereinrichtung, der Entfernungseinrichtung und der Bestimmungseinrichtung, so daß sich die Konzentrationsänderung des das Gewebe beeinflussenden chemischen Stoffes in einer im wesentlichen linearen Weise ändert und zu jedem Zeitpunkt bekannt ist.

11. Vorrichtung nach Anspruch 10, dadurch gekennzeichnet, daß die Einrichtung zum Steuern des Betriebs der Addiereinrichtung, der Entfernungseinrichtung und der Bestimmungseinrichtung ein programmierter Ditigalrechner ist.

12. Vorrichtung nach Anspruch 10, ferner gekennzeichnet durch eine Einrichtung zum Analysieren ausgewählter erster bekannter Mengen des chemischen Stoffes, die aus dem ersten, das Gewebe beeinflussenden Volumen entfernt wurden;

eine Einrichtung zum Anpassen der ausgewählten, analysierten Mengen des chemischen Stoffes an chronologisch entsprechende, dritte bekannte Konzentrationen des das Gewebe beeinflussenden chemischen Stoffes, um eine Beziehung der Antwort des Gewebes bezüglich ausgewählter Konzentrationen des chemischen Stoffes aufzustellen; und

eine Einrichtung zum Kombinieren der einzelnen Antworten und der entsprechenden Konzentrationen des chemischen Stoffes zum Bilden einer Beziehung, die die Reaktion des Gewebes auf die Konzentrationsänderung des chemischen Stoffes über die Zeit darstellt.

**Revendications**

1. Procédé pour délivrer une substance chimique en un programme prédéterminé de variation de concentration à un tissu biologique, selon lequel on fait varier la concentration de la substance chimique sur une période totale de temps, le procédé comprenant:

(a) le choix d'un programme de variation de concentration de la substance chimique s'étendant et variant sur la totalité de la période de temps;

(b) le scindage de la période totale de temps répartie en plusieurs périodes plus petites de temps agencées chronologiquement, chaque période plus petite de temps étant dépourvue de tout point d'inflexion dans le programme de variation de concentration et ayant un niveau de concentration initiale et un niveau de concentration finale de la substance chimique;

(c) le choix d'un débit de délivrance de la substance chimique et d'une concentration de substance chimique délivrée dans la période plus petite de temps selon une courbe de concentration définie par

$$C_{(t)}=C_s+(C_i-C_s)e^{-Rt/V}$$

dans laquelle

$t$=la période plus petite de temps

$V$=volume de fluide au sein d'une chambre affectant la cellule ou le tissu dans lequel la substance chimique est délivrée

$R$=le débit de délivrance de la substance chimique à la chambre

$C_i$=le niveau de concentration initiale de la substance chimique dans la période plus petite de temps au sein de la chambre

$C_{(t)}$=la concentration finale de la substance chimique dans la période plus petite de temps au sein de la chambre

$C_s$=la concentration de la substance chimique délivrée au cours de la période plus petite de temps à partir d'un courant d'alimentation dans la chambre,

et qui est approximativement linéaire et comprend $C_i$ et $C_{(t)}$ par le choix des valeurs de $R/V$ et de $C_s$;

(d) la fourniture d'une source d'alimentation contenant la substance chimique ayant une concentration supérieure à $C_s$ pour chaque période plus petite de temps;

(e) la délivrance de la substance chimique de la source de fourniture à la chambre au débit $R$ et à la concentration $C_s$, comme défini en (c);

(f) la répétition de (c) à (e) pour chaque période plus petite de temps subséquente de manière que le programme choisi permette essentiellement d'obtenir la réponse de la cellule ou du tissu à la délivrance de la substance chimique selon le programme de variation de concentration.

2. Procédé selon la revendication 1, dans lequel la cellule ou le tissu se trouve dans une chambre de culture et dans lequel la chambre à laquelle est délivrée le niveau de concentration $C_s$ se trouve

en communication de liquide avec la chambre de culture.

3. Procédé selon la revendication 1, dans lequel la chambre est une chambre de culture contenant le tissu.

4. Procédé selon la revendication 1, dans lequel le tissu est un organe et la délivrance de la substance chimique s'effectue de la chambre à l'organe.

5. Procédé selon la revendication 1, comprenant en outre l'étape consistant à:

réaliser les étapes (b) et (c) à l'aide d'une calculatrice numérique programmée.

6. Procédé selon la revendication 1, comprenant en outre la délivrance de la substance chimique à une concentration constante à une cellule ou à un tissu semblable dans une autre chambre pour une comparaison avec la réponse de la cellule ou du tissu à la délivrance de la substance chimique selon la revendication 1.

7. Procédé selon la revendication 1, dans lequel le tissu biologique est une cellule.

8. Procédé selon la revendication 1, dans lequel le tissu est isolé en étant placé dans une chambre de culture comportant une admission par laquelle la substance chimique est fournie et une sortie par laquelle la substance chimique est enlevée.

9. Procédé selon la revendication 1, dans lequel plusieurs substances chimiques sont délivrées au tissu biologique, chacune en son programme prédéterminée propre de variation de concentration, selon le procédé décrit à la revendication 1.

10. Appareil pour délivrer une substance chimique en un programme de variation prédéterminé de concentration à un tissu biologique par un procédé selon l'une quelconque des revendications 1 à 9, en commençant par un premier volume connu de la substance chimique ayant une concentration connue affectant le tissu, l'appareil comprenant:

a) un moyen pour enlever une première quantité connue de la substance chimique pour qu'elle n'affecte plus le tissu, en laissant un second volume connu ayant une seconde concentration connue affectant le tissu;

b) un moyen pour ajouter une seconde quantité connue de la substance chimique, ayant une concentration différente, au second volume connu affectant le tissu, en formant ainsi un troisième volume connu affectant le tissu;

c) un moyen pour déterminer une troisième concentration connue du troisième volume connu de la substance chimique affectant le tissu, sur la base de l'enlèvement de la première quantité connue et de l'addition de la seconde quantité connue; et

d) un moyen pour commander le fonctionnement du moyen destiné à ajouter, du moyen destiné à enlever et du moyen destiné à déterminer de façon que la variation de concentration de la substance chimique affectant le tissu change d'une manière essentiellement linéaire et est connue à chaque instant.

11. Appareil selon la revendication 10, dans lequel le moyen pour commander le fonctionnement du moyen pour ajouter, du moyen pour enlever et du moyen pour déterminer, est une calculatrice numérique programmée.

12. Appareil selon la revendication 10, comprenant en outre:

un moyen pour analyser des premières quantités connues choisies de la substance chimique enlevée du premier volume affectant le tissu;

un moyen pour faire correspondre les premières quantités analysées et choisies de la substance chimique avec des troisièmes concentrations connues, chronologiquement correspondantes, de la substance chimique affectant le tissu pour établir une relation entre la réponse du tissu et les concentrations choisies de la substance chimique; et

un moyen pour combiner les réponses individuelles et les concentrations correspondantes de la substance chimique pour former une relation établissant la réaction du tissu à une variation de concentration de la substance chimique en fonction du temps.

Administered
Dose

Drug Concentration

Desired
Dose

TIME
PRIOR ART
FIG.1

Administered
Dose

Drug Concentration

Desired
Dose

TIME
PRIOR ART
FIG. 2

Drug Concentration

TIME
FIG. 3

Drug Concentration

.X.

First
Pulse

Second
. Pulse

TIME
PRIOR ART
FIG. 4

Substance A
Substance B

Drug Concentration

TIME
FIG. 5

FIG. 6

FIG. 7

FIG. 7A

FIG. 8

22
24
20
26
28
30
32

22
24
20
26
28
Artery Organ Vein
34

HOLDING FLASK #1
HOLDING FLASK #2
HOLDING FLASK #3

VALVE

PUMP

CULTURE CHAMBER

pH
O2
TEMPERATURE
SELECTED IONS
MEMBRANE POTENTIALS

PROGRAMMED DIGITAL COMPUTER CONTROL SYSTEM

OUTPUT DEVICE

INPUT DEVICE

COLLECTOR

ANALYZER

0 101 715

FIG. 9
r=Correlation Coefficient

r=0.99989     r=0.99993

FIG. 10
R= Rate of Delivery
V= Volume Affecting Tissue

FIG.11A

FIG.11B

FIG. 12
Intact Ovary
Pulsatile
FOLLICULAR DISTRIBUTION
Static

0 101 715

FIG. 13

FIG. 14

FIG. 15

FIG. 16

5